# EUROPEAN PATENT SPECIFICATION

(11) **EP 1 521 826 B1**
(45) Date of publication and mention of the grant of the patent: **11.07.2007**
(21) Application number: 02743540.3
(22) Date of filing: 04.07.2002
(51) Int. Cl.: C12N 13/00, C12N 15/00

(54) **A SIMPLE, EFFICIENT, AND ACCELERATED METHOD FOR ENZYME-CATALYZED IN VITRO MODIFICATION AND SYNTHESIS OF NUCLEIC ACID USING MICROWAVE IRRADIATION**
EINFACHES, EFFIZIENTES UND BESCHLEUNIGTES VERFAHREN ZUR ENZYM-KATALYSIERTEN IN VITRO MODIFIZIERUNG UND HERSTELLUNG VON NUKLEINSÄUREN UNTER VERWENDUNG VON BESTRAHLUNG IM MIKROWELLENBEREICH
PROCEDE SIMPLE, EFFICACE ET ACCELERE DE MODIFICATION IN VITRO CATALYSEE PAR UNE ENZYME ET DE SYNTHESE D'ACIDE NUCLEIQUE UTILISANT UNE IRRADIATION PAR HYPERFREQUENCES

(43) Date of publication of application: 13.04.2005
(73) Proprietor: COUNCIL OF SCIENTIFIC AND INDUSTRIAL RESEARCH, New Delhi 100 001 (IN)
(72) Inventor: DAS, Rakha, Hari, Delhi 110 007 (IN); NAHAR, Pradip, Delhi 110 007 (IN)
(74) Representative: Jostarndt, Hans-Dieter
(86) International application number: PCT/IB2002/002766
(87) International publication number: WO 2004/005505

(56) References cited:
- WO-A-93/21344
- WO-A-95/15671
- WO-A-98/06876
- KAPPE C OLIVER: "High-speed combinatorial synthesis utilizing microwave irradiation." CURRENT OPINION IN CHEMICAL BIOLOGY, vol. 6, no. 3, June 2002 (2002-06), pages 314-320, XP002238125 ISSN: 1367-5931
- PARKER M-C ET AL: "Microwave Radiation Can Increase The Rate of Enzyme-Catalysed Reactions in Organic Media" TETRAHEDRON LETTERS, ELSEVIER SCIENCE PUBLISHERS, AMSTERDAM, NL, vol. 37, no. 46, 11 November 1996 (1996-11-11), pages 8383-8386, XP004068668 ISSN: 0040-4039

## Description

### Technical Field

The present invention relates to a simple, efficient, and accelerated method for enzyme-catalyzed in *vitro* modification and synthesis of nucleic acid using uninterrupted and brief microwave irradiation at a frequency ranging between 2300 to 2500 MHz, with the power output ranging between 600 to 900 watts, and for a period ranging from 5 to 120 seconds; and further, an apparatus for using said method.

### Background Art

Enzymatic reactions of nucleic acids are key to molecular biologists. The most commonly used enzymatic reactions in molecular biology are site specific cleavage, ligation, dephosphorylation, phosphorylation (kination) and *in vitro* DNA and RNA synthesis reactions. These reactions are carried out by different enzymes such as restriction endonuclease; ligase, phosphatase, kinase, DNA. polymerases, reverse transcriptase and RNA polymerase. These reaction products have a wide range of applications in molecular biology.

The discovery of several restriction endonucleases (RENs) gave birth of modern biotechnology. These enzymes catalyze the sequence specific hydrolytic cleavage of double-stranded DNA by nicking in the opposite strands.

Ligase is another important enzyme, which can modify the nucleic acids by the formation of phosphodiester bond between two compatible ends of double-stranded DNA and single-stranded nicks in DNA-DNA or DNA-RNA hybrid. [Engler, M.J. and Richardson, C.C. (1982) in The Enzymes (Boyer, P.D, ed) Vol.5, p.3, Academic Press, San Diego]. The products of DNA ligase catalyzed reactions are required for cloning and manipulation of gene.

Phosphatase catalyzes the removal of terminal phosphate groups of nucleic acids [Sambrook, J., Fritsch, E.F. and Maniatis, T. (1989) Molecular cloning, A Laboratory Manual, second edition. Cold Spring Harbor Laboratory, Cold Spring Harbor, New York] and is necessary to reduce the vector background in cloning and end labeling with [γ ³²P] ATP.

Polynucleotide kinase modifies the nucleic acids by transferring or exchanging the phosphate group from the gamma position of ATP to the 5' hydroxyl terminus of polynucleotides (double- and single-stranded DNA and RNA) and nucleoside 3'monophsphates [Sambrook, J., Fritsch, E.F. and Maniatis, (1989) Molecular cloning, A Laboratory Manual, second edition, Cold Spring Harbor Laboratory, Cold Spring Harbor, New York].

The template directed synthesis of DNA and RNA catalyzed by respective polymerase is routinely carried out in recombinant DNA research. The DNA template directed DNA synthesis is used in preparing radio labeled hybridization probes by nick translation [Rigby et al. (1977), J. Biol. Chem. 113: 237], end filling reactions for ligation and labeling of DNA fragments, second strand cDNA synthesis, polymerase chain reaction (PCR). DNA sequencing etc.

The RNA template directed DNA synthesis catalyzed by reverse transcriptase is required for the synthesis of cDNA and amplification of gene by RT-PCR. The DNA template directed RNA synthesis (transcription) is utilized for identification of RNA polymerase specific promoter, coupled transcription and translation reactions and making highly labeled RNA probes etc.

Traditionally the reactions are carried out by incubating in water bath maintained at the optimum temperature at which the enzyme exhibits maximum activity. The incubation time is varied from minutes to hours depending on the particular enzyme and the substrate as well as their concentrations. Most of these reactions are often required to be carried out in series to achieve a particular objective, as the product (s) of one reaction is the substrate for the another. Even though most of these reactions take few minutes to hours the overall time consumed to complete a particular set of reaction is quite high. It is generally preferred to give longer reaction time to ensure the completion of reaction.

Main drawback of the traditional procedures is the long reaction time and the molecular biologists have to spend much of their precious time to perform the time consuming experiments with these enzymes. One of the ways to make the enzyme-catalyzed reaction faster is either to activate the enzyme or the substrate or both. Organic reaction usually is accelerated by thermal heating, which is not always possible in case of enzymes as most of them are susceptible to heat inactivation. One of the alternate ways to accelerate a chemical or biological reaction is to use microwaves as energy source. In fact, the use of microwaves for accelerating the rate of organic reactions was known for a long time.

In 1986, Gedbye et. al and Giguere et. al demonstrated that many organic reactions could be conducted very rapidly by microwave irradiation [Gedye, R.N. et. al., Tetrahedron Lett. (1986), 26, 279; Giguere, R.J et. al., Tetrahedron Lett. (1986), 27, 4945-4948]. Since then versatility of "Microwave- induced Organic Reactions Enhancement' (MORE) chemistry was being demonstrated by many authors in a variety of organic reactions such as catalytic hydrogenation, Diels-Alder reaction, free radical reaction, acylation, deacylation etc. *[*Bose.A.K, et al., Res. Chem. Intermed. (1994) 20, 1-11; Bose.A.K et al., J.Org. Chem. (1991) 56, 6968-6970; Nahar, P. Tetrahedron Lett. (1997), 38, 7253-7254]. Practically all these reactions were carried out in a domestic microwave oven, operating at a frequency of 2450 MHz and maximum power output is around 700 watts. These ovens are usually have 10 different levels corresponding to the different power output, level ten having the maximum or highest power output whereas level one has lowest power output of the microwave oven. Although drastic reduction in time is the main advantage of microwave reactions, the selectivity or specificity of the reactions were also being reported [Nahar, P. Tetrahedron Lett. (1997), 38, 7253-7254].

Microwave energy has also been used successfully for protein assay [Akins, Jr. et al.; U.S. patent nos. 5,403,747 and 5,478,748] and immunohistochemistry [Boon, M.E. et. al; Am. J. Clin. Pathol. (1989), Boon, M.E. et. al; In Microwave Cookbook of Pathology : The art of Microscopic Visualisation]. Hydrolysis of adenosine triphosphate by microwave irradiation has also been studied [Jahngen, E.G.E. et.al. J.Org. Chem. (1990) 55, 3406-3409].

The effect of microwave irradiation on biopolymers, especially on enzymes and DNA has been studied by several workers. However, there were contradictory reports on the effect of microwave irradiation on the biomolecules. In some cases, microwaves were used to inactivate brain enzymes of small laboratory animals [Galli Claudio and Racagni Giogio, Methods in enzymology, (1982) 86, 636-642]. However, Byus, C.V. et al., have found an increase in ornithine decarboxylase activity in cultured cells using microwave energy [Byus et al, Cancer Res. (1988) 48,4222-4226]. There are also reports where enhancement as well as repression of cellular enzymatic activity was observed [Dutta, S.K. and Verma, M. Current Scince (1989) 58, 58-63; Dutta, S.K. and Verma, M. Current Scince (1988) 57, 779-786]. Maleev, V.Ya. et. al could not find any effect of enhanced microwave absorbtion by DNA (Maleev, V.Ya et al. Biopolymers (1987) 26, 1965-1970). However, according to Narasimhan et al. DNA molecule can exhibite single-strand break, double strand-break, localized strand separations etc when exposed to microwaves [Narasimhan, V. and Huh, W.K.; Biochem. International (1991) 25, 303-370).

Enhancement of enzymatic reactions for nucleic acid synthesis and modifications by microwaves is not reported earlier although the nucleic acid digestion by restriction enzymes was reported by Jhingan (U.S. Pat. No. 5,350,686). The author digested DNA by restriction enzyme using microwave energy at a lowest power level that is 10% of the out put power of 700 watt of a standard microwave oven. During the reaction author also used a series of intervals, in which microwave power is not applied to the reaction mixture. In this method the total reaction time for digestion reactions with most of the enzymes were between 6 to 15 minutes. However, some of the control samples of plasmid DNA when incubated at 37°C showed nearly complete digested products. From this experiment it is not clear how much microwave irradiation was responsible for the above enzymatic reactions as most of the reaction time enzyme did not get proper microwaves. It seems that the time has played a major role than the microwaves in these cases.

The reported method (U.S. Pat. No. 5,350,686) for microwave mediated enzyme catalyzed modification of macromolecules has several drawbacks such as (1) time gain was not attractive. It may be possible to get comparable results for some of the restriction enzyme digestion reaction out side microwave oven in the same time at 37°C, (2) the procedure require a series of intervals in which microwave power is not applied to the reaction mixture, (3) there was no uniform condition for enzymatic reactions. In most of the cases author used different microwave exposure time for digestion reaction using restriction enzyme and (4) automation of the procedure will be not very effective due to (a) longer reaction time and (b) ununiformity of reaction conditions.

The applicants in the present invention have overcome all the above drawbacks. For any reaction an optimum condition is required. Optimum thermal energy accelerates an enzymatic reaction, in mild condition (below optimum condition) the reaction slows whereas in drastic condition (above optimum condition) enzyme deactivates. The same is true for reactions using microwave energy. If optimum conditions are not maintained enzymatic reactions may be spoiled or give incomplete reaction product. In the present invention, proper conditions were found most of, which are contrary to the reported method, or not known in the prior art.

More over, enzymatic reaction by microwaves greatly depends on the composition of the reaction mixture apart from enzyme and the substrate. Buffer, its pH, salt concentration and other ingredients play a, major role in enzymatic reactions, more particularly when it is done by microwaves. We have invented that in dilution buffer, containing 50% (v/v) glycerol, the restriction enzyme was inactivated when exposed to continuous microwaves at high power, even for a short period (15 seconds and above) whereas in conventional reaction at 37°C it remained active. Dilution buffer is recommended by the supplier for diluting the high concentration enzyme when required.

Contrary to the work reported by Jhingan (U.S. Pat. No. 5,350,686), we have now invented that enzymatic modification of a biological macromolecule could be carried out at the highest power setting of a microwave oven operating at a frequency of 2450 MHz and a power of 700 watts. More over, we also found that there is no need of any break during the microwave mediated enzymatic reactions, which is again contrary to the above work.

### Objectives of the invention

The main objective of the present invention is to provide a rapid and efficient method for enzymatic reactions to produce specific products of these enzyme-catalyzed reactions that are essential for analysis and manipulation of genes in molecular biology and recombinant DNA technology and other fields.

Another objective of the present invention is to provide a method, which is simple, reproducible and does not require additional expertise or costly equipment.

Yet another objective of the present invention is to provide a method that can be automated in a simple way for carrying out most of the enzymatic reactions used in biotechnology or molecular biology in a single instrument.

Still another object of the present invention is to develop a method involving irradiation that does not lead to adverse effect on the enzyme.

### Summary of the invention

The present invention relates to a simple, efficient, and accelerated method for enzyme-catalyzed in *vitro* modification and synthesis of nucleic acid using uninterrupted and brief microwave irradiation at a frequency ranging between 2300 to 2500 MHz, with the power output ranging between 600 to 900 watts, and for a period ranging from 5 to 120 seconds; and further, an apparatus for using said method.

### Detailed description of the present invention

Accordingly, the present invention relates to a simple, efficient, and accelerated method for enzyme-catalyzed in *vitro* modification and synthesis of nucleic acid using uninterrupted and brief microwave irradiation.

In an embodiment of the present invention, providing a reaction mixture comprising (i) an enzyme selected from the group consisting of restriction endonuclease, ligase, phosphatase, kinase, reverse transcriptase, DNA polymerase and RNA polymerase (ii) a biomolecule selected from DNA, RNA or oligonucleotides, to act as a substrate for the selected enzyme, (iii) suitable buffer for specific enzyme and (iv) other ingredients, in a small eppendorf tube or on a small piece of parafilm

In another embodiment of the present invention, placing the said eppendorf tube or parafilm piece, loaded with the reaction mixture, inside a microwave oven.

In yet another embodiment of the present invention, irradiating the said eppendorf tube or parafilm by microwaves continuously at a frequency ranging between 2300 to 2500 MHz with the power output ranging between 600 to 900 watts.

In still another embodiment of the present invention, for a period ranging from 5 to 120 seconds.

In still another embodiment of the present invention, terminating the reaction by adding ethylenediamine tetra acetate (EDTA) and/ or heating in a water bath at 75°C for 3-15 minutes followed by the addition of gel loading dye.

In still another embodiment of the present invention, analyzing the reaction product (s) by agarose gel electrophoresis, autoradiography, radioactive counts and other techniques as used in the conventional procedures.

In still another embodiment of the present invention, wherein nucleic acid is selected from DNA, RNA, and oligonucleotide.

In still another embodiment of the present invention, wherein other ingredients used are selected from a group comprising MgCl₂, MgSO₄, NaCl, KCl, CH₃COOK, ethylenediamine tetra acetate, dithiothreitol, spermidine, BSA, triton x-100, nucleotides, template DNA, template RNA, and oligonucleotide primers.

In still another embodiment of the present invention, wherein restriction endonuclease is selected from Hind III, BamHI, EcoRI, Hae III, Bgl II, Pst I, Bst E II, and Bst NI.

In still another embodiment of the present invention; wherein ligase used is T4 DNA ligase.

In still another embodiment of the present invention, wherein kinase used is T4 polynucleotide kinase (TPNK).

In still another embodiment of the present invention, wherein phosphatase used is calf intestinal alkaline phosphatase (CIP).

In still another embodiment of the present invention, wherein polymerase is selected from the group consisting of E.coli. DNA polymerase I, Klenow fragment of E.coli. DNA polymerase 1, T7 RNA polymerase, and SP6 RNA polymerase.

In still another embodiment of the present invention, wherein reverse transcriptase used is avian myeloblastosis virus-reverse transcriptase (AMV-RT).

In still another embodiment of the present invention, wherein DNA used is selected from the group comprising genomic DNA, lambda phage DNA, plasmid DNA, baculovirus DNA, plant DNA, and mammalian DNA.

In still another embodiment of the present invention, wherein more than one restriction endonuclease is used for double digestion of nucleic acid.

In still another embodiment of the present invention, wherein restriction endonucleases used are EcoRI and Hind III for double digestion.

In still another embodiment of the present invention, wherein microwave irradiation can be carried out in any apparatus or chamber where microwave can be generated.

In still another embodiment of the present invention, wherein the said method can be partially of fully automated by a specially designed apparatus.

In still another embodiment of the present invention, an apparatus suitable for carrying out the enzymatic reaction as stated above.

In still another embodiment of the present invention, a reaction chamber consisting of magnetron, exhaust fan, fiber optic thermometer, cooling system, and rotating reaction platform.

In still another embodiment of the present invention, a microprocessor based computing means for giving preprogrammed command for carrying out different reactions through suitable hardware and software.

In still another embodiment of the present invention, wherein magnetron is the source of microwaves, operated at the frequency of about 2450 MHz with the power output ranging from 100 watts to 800 watts.

In still another embodiment of the present invention, this invention relates to a rapid and efficient method for the site specific cleavage, modification and synthesis of biological molecules. More particularly, production of designed biomolecules by enzyme-catalyzed reactions using brief and uninterrupted microwave irradiation is described. The invented procedure is adaptable in biotechnology, more particularly in molecular biology, enzyme based industrial processing and sewage treatment and also suitable for automation in the related field.

In still another embodiment of the present invention, this invention particularly relates to a rapid and efficient method for site specific cleavage, modification, and *in vitro* synthesis of biomolecules by microwave enhanced enzymatic reactions. This invention particularly relates to a quick and efficient procedure to carryout the enzyme-catalyzed site specific cleavage, modification and in *vitro* synthesis of nucleic acids by brief and continuous microwave irradiation without any break. The preferred microwave frequency is from 2000 to 2800 MHz with power out put of from 500 to 900 watts. The preferred microwave irradiation time is from 5 to 120 seconds. The cleavage, modification and synthesis of nucleic acids are obtained in reactions more particularly catalyzed by restriction endonuclease, ligase, phosphatase, kinase, reverse transcriptase, DNA polymerase and RNA polymerase. The specific products of these enzyme-catalyzed reactions are essential for analysis and manipulation of genes in molecular biology and recombinant DNA technology.

In still another embodiment of the present invention, this invention relates to a rapid method for the preparation of enzyme-catalyzed reaction products. This invention, particularly relates to a quick and efficient procedure to carry out the enzyme catalyzed site specific cleavage, modification and *in vitro* synthesis of nucleic acids. The synthesis, modification and cleavage of nucleic acids are obtained in reactions more particularly catalyzed by restriction endonuclease, ligase, phosphatase, kinase, DNA polymerase, reverse transcriptase and RNA polymerase. This is a very rapid method in carrying out site specific cleavage, ligation, dephosphorylation, phosphorylation (kination) and *in vitro* DNA and RNA synthesis reactions. The specific products of these enzyme-catalyzed reactions are essential for analysis and manipulation of genes in molecular biology and recombinant DNA technology.

In still another embodiment of the present invention, with a view to achieve the objective of the present invention and overcoming the disadvantage of the known method for the enzymatic reaction, a rapid and efficient method is provided for the site specific cleavage, modification and *in vitro* synthesis of nucleic acids by microwave mediated enzymatic reactions. The enzymatic reactions that are used in molecular biology, namely site specific cleavage, ligation, dephosphorylation, phosphorylation (kination), *in vitro* DNA synthesis and *in vitro* RNA synthesis were carried out in the present invention.

In still another embodiment of the present invention, the present invention comprises (a) subjecting the reaction mixture containing the enzyme, substrate, suitable buffer and other ingredients (as described in the examples) to microwave exposure in the microwave oven at the highest power out put of the microwave oven (700watts) for a short period without any break.

In still another embodiment of the present invention, the procedure results in the site-specific cleavage, modification and *in vitro* synthesis of nucleic acids in 5 to 120 seconds. The enzymatic reactions carried out by the invented procedure showed reproducible results making the procedure versatile. This new finding for cleavage, modification and synthesis of macromolecules, more particularly nucleic acids in less than 140 seconds by the exposure of brief and uninterrupted microwaves to the enzyme - substrate reaction mixture is disclosed.

In still another embodiment of the present invention, this is very rapid method known so far for the enzyme-catalyzed site-specific cleavage, modification and *in vitro* synthesis of nucleic acids. The *in vitro* syntheses in the invented procedure include DNA template directed DNA and RNA syntheses and RNA template directed DNA synthesis (reverse transcription). The method has the potential for automation.

In still another embodiment of the present invention, novelty of the present invention is that most of the reactions are completed within 5-150 seconds, where microwave effect is clearly demonstrated against the time effect.

In still another embodiment of the present invention, another novelty of the invention is the finding that restriction enzymes in dilution buffer containing 50% (v/v) glycerol lost its enzymatic property when exposed to microwave energy even for a short time at high power out put.

In still another embodiment of the present invention, another novelty of the invention is that the restriction enzymes in digestion buffer remained active when exposed to microwave energy at high power out put (700 watts) for a short time (at least for 140 seconds) without any break.

In still another embodiment of the present invention, another novelty of the present invention is that the reaction conditions are more uniform contrary to the reported method where in most of the cases, digestion of DNA by different restriction enzymes required different reaction conditions.

In still another embodiment of the present invention, another novelty of the invention is that the all most all the enzymatic reactions, commonly used in molecular biology can be carried out by the invented method.

In still another embodiment of the present invention, another novelty of the invention is that the invention can be automated in a simple way for carrying out most of the enzymatic reactions used in molecular biology in a single instrument.

In still another embodiment of the present invention, the present invention provides a rapid and efficient method for the site specific cleavage, modification and *in vitro* synthesis of nucleic acids by microwave induced enzymatic reactions, namely site specific cleavage, ligation, dephosphorylation, phosphorylation (kination) *in vitro* DNA synthesis and in *vitro* RNA synthesis. The procedure comprises subjecting the reaction mixture containing the enzyme to microwave exposure in the microwave oven at the highest power level for a short period without any break. Enzymatic reactions are delicate method where improper conditions can hamper the results.

In still another embodiment of the present invention, the inventors have found enzymatic activity after microwave exposure at the highest power level of a microwave oven (700 watts) for a brief period ranging from 5 to 120 seconds. Our findings are in contrary to the report (U.S. Pat. No. 5,350,686). where deactivation of the enzymes were found after microwave exposure at a much lower power level. This may be due to the fact that prior art used much longer irradiation time ranging from 15 to 31 minutes with different number of intervals. The inventors carried out the enzymatic reactions by microwave irradiation at the highest power level for a brief period without any break.

### Detailed description of the accompanying drawings

### Fig. 1.

### Stability of Hind III on microwave exposure at 700 watts.

HindIII in its digestion buffer was exposed to microwaves for different periods: 0 (lane 1), 30 (lane 2), 50 (lane 3), 70 (lane 4), 90 (lane 5), 110 (lane 6) and 130 (lane7) seconds. HindIII irradiated for different time periods were assayed by digestion of lambda DNA by exposing them to microwaves at 700 watts for 50 seconds. In each lane of the 1.0, % agarose gel, 1.0 µg of digested DNA was loaded (example 1).

### Fig. 2

### Restriction endonuclease digestion of DNA by microwave irradiation at 700 watts.

Digestions of lambda DNA by Hind III (lane2), BamHI (lane 3), EcoRl (lane 4), double digestions of lambda DNA by EcoRI and HindIIII (lane 5) and digestion of a pea lectin cDNA clone at the PstI site of PUC19 vector by PstI (lane 6) were carried out using 2.0 µg of DNA, 1.0 µl of each enzyme in their respective digestion buffer in 20.0 µl reaction volume. Double-digestion with HindIII and EcoRI was done in EcoRI digestion buffer. In the control reaction (lane 1) 2.0 µg lambda DNA in 20.0 µl HindIII digestion buffer without enzyme was used. In each lane of the 1.0 % agarose gel, 1.0 µg DNA was loaded (example 2).

### Fig. 3

### T4 DNA ligase catalyzed ligation of lambda HindIlI fragments and fragmentation of ligated DNA by HindIII in the microwave oven.

Lane 1 was loaded with uncut lambda DNA (1.0 µg) irradiated to microwaves for 50 seconds in HindIII digestion buffer. Lane 2 represents HindIII cut lambda DNA (1.0 µg) prepared by exposing the reaction mixture to microwaves for 50 seconds. Lane 3 is loaded with the products obtained in the ligation,reaction of HindIII cut lambda DNA, carried out at 37°C for 45 minutes. Lane 5 was loaded with the products obtained in the ligation reaction of HindIII cut lambda DNA, carried out by microwave irradiation for 20 seconds. Ligated DNAs as obtained in lane 3 and 5 were re-cut with HindIII by microwave irradiation for 50 seconds and were loaded in lanes 4 and 6 respectively of the 1.0 % agarose gel. Each of these lanes contained 1.5 µg DNA (example 3).

### Fig. 4

### Kination of 60 mer oligonucleotide by T4 polynucleotide kinase.

Kination of 60 mer oligonucleotide (5' - CCCCGCCCCGCG - 3') ₅ by T4 polynucleotide kinase on exposure to microwaves for 20 (lanel), 30 (lane 2), 50 (lane 3) and 0 (lane 4) seconds (example 5 b)

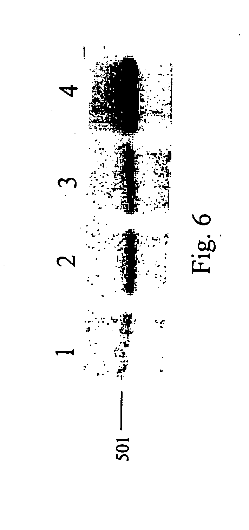

### Fig. 5. Lane 1.

### Microwave mediated DNA synthesis by Klenow fragment E. coli. DNA polymerase I.

Autoradiogram of 1 % agarose gel of the products produced by HindIII digestion of lambda DNA, followed by end filling reaction by *E*. *coli.* DNA polymerase I. Both the reactions were carried out by microwave irradiation at 700 watts for 50 and 20 seconds respectively (example 7)

### Fig. 5. Lane 2.

### Microwave mediated digestion, dephosphorylation and kination reactions.

TPNK catalyzed kination of CIP dephosphorylated EcoRI fragments of lambda DNA. Autoradiogram of 1 % agarose gel of the products produced by EcoRI digestion of lambda DNA followed by dephosphorylation of 5'-ends of EcoRI fragments by CIP and kination of dephosphorylated 5'-ends of EcoRI fragments by TPNK (example 5a).

### Fig. 6

### Microwave mediated E-selectin mRNA template directed cDNA synthesis by AMV-RTase.

A 501 bp specific cDNA was obtained when AMV-RTase catalyze reaction products obtained in 10 (lane 1), 30 (lane 3) and 50 (lane 4) seconds by microwave irradiation and in 45 min at 48°C (lane 2) were PCR amplified using Tfl 1 DNA polymerase. RT-PCR reaction products were analyzed in 1.4% agarose gel (example 8).

### Fig.7

### Microwave mediated synthetic mRNA template directed cDNA synthesis by AMV-RTase.

A 323 bp specific cDNA was obtained when AMV-RTase catalyze reaction products obtained in 30 (lane 5) and 50 (lane 6) seconds by microwave irradiation and in 45 min at 48°C (lane 3) were PCR amplified using Tfl 1 DNA polymerase. RT-PCR reaction products were analyzed in 1.4 % agarose gel. In control reactions, lane 2 (45 min at 48°C) and lane 4 (50 s microwave exposure) RNA template (an 1151 nt *in vitro* synthesized RNA from Promega) was not added. In lane 1, pBR 322 BstN1 marker DNA was loaded (example 9).

**TABLE -1**

| **Comparison between the prior art and the present invention and the advantages of the present invention over the prior art.** | | |
|---|---|---|
| | Prior Art | Present Invention |
| | (U.S patent no. 5,350,686) | |
| 1. | Accelaration of restriction enzyme- catalyzed site-specific cleavage of DNA by interupted microwave irradiation at 70 watts. | In vitro synthesis of nucleic acids, site specific cleavage, ligation, kination, and dephosphorylation of DNA are carried out by continuous microwave irradiation at 700 watts using the respective enzymes. |
| 2. | Cleavage reaction took 2-24 minutes. | All the reactions are very fast. Only 5-120 seconds are required for the completion of the reaction. |
| 3. | Non uniform reaction conditions. Different restriction enzymes required different microwave exposure and nonexposure time. | Uniform reaction conditions. Brief and continuous microwave irradiation. Same conditions applied for most of the reactions of the same type, such as cleavage, kination etc. |
| 4. | Method less suitable for automation as time gain is not attractive, reaction conditions varied for the cleavage reaction by different enzymes of the same type. | Method more suitable for automation as time gain is very attractive and has general applicability. It can apply to most of the reactions used in molecular biology. |
| 5. | Restriction enzyme lost its activity at high power setting. Hence, enzymatic reaction with DNA was not carried out by microwave irradiation at high power (700 Watts). | Restriction enzyme in dilution buffer lost its activity when exposed to continuous microwave irradiation at 700 watts, even for a brief period (above 20 seconds). How ever, in a breakthrough finding it was invented that restriction enzyme remained active in digestion buffer when exposed to microwaves at high power (700 watts) for a brief period (atleast for 130 seconds). This finding has led to the present invention where synthesis, modification and cleavage of nucleic acid using the respective enzyme were carried out by brief and continuous microwave irradiation at 700 watts. |
| 6. | Restriction enzyme catalyzed site-specific cleavage of DNA was carried out by interrupted microwave irradiation at 70 watts. No other enzymatic reaction was carried out. | Different reactions commonly used in molecular biology such as restriction endonuclease digestion of DNA, ligation by T4 DNA ligase, dephosphorylation by alkaline phosphatase, kination by T4 polynucleotide kinase, DNA syntheses by E. Coli polymerase I, reverse transcriptase and RNA synthesis by T4 RNA polymerase were carried out by continuous and brief microwave irradiation at 700 watts. |
| 7. | Except DNA no other molecule is used as a substrate in the enzymatic reaction. | Oligonucleotide was also used as a substrate. |
| 8. | Time gain was not attractive. It may be possible to get comparable results for some of the restriction enzyme digestion reaction out side microwave oven in the same time at 37°C. | Novelty of the present invention is that most of the reactions are completed in seconds, where microwave effect is clearly demonstrated against the time effect. |
| 9. | The method is not rapid, simple, uniform and universal. | The method is rapid, simple, uniform and universal. |
| 10. | The product obtained in the prior art was specifically cleaved DNA | The products obtained in the prior art are DNA, modified DNA, RNA, modified oligonucleotide or oligonucleotide. |
| 11. | Generally in molecular biology a series of (more than one) reactions are to be performed , to achieve a particular objective. Prior art gave an example of only one type of reaction that is site specific cleavage of DNA. | As the present invention teaches the method for different enzymatic reactions commonly used in molecular biology by brief and uninterrupted microwave exposure, therefore the present invention has more advantages to carry out a series of experiments in short time. |

In still another embodiment of the present invention, the present invention comprises a rapid and efficient method for site specific cleavage, modification and *in vitro* synthesis of nucleic acids by microwave induced enzymatic reactions, namely restriction endonuclease digestion, ligation, dephosphorylation, phosphorylation (kination), *in vitro* DNA synthesis, reverse transcription and *in vitro* RNA synthesis. The procedure comprises subjecting the reaction mixture containing the enzyme and the substrate to microwave exposure in the microwave oven at its highest power output for a short period without any break.

In still another embodiment of the present invention, the procedure results in a highly selective specific cleavage, modification and synthesis of nucleic acids in 5 to 120 seconds. All the reactions were carried out for different duration of microwave exposure time to find the optimum reaction time. The products produced by the respective enzyme on microwave irradiation of their reaction mixtures were identical to that produced by the conventional procedures carried out at optimum temperature (37°C or below) for 30 minutes to several hours. Control reactions carried out outside microwave oven for the same time (as used in microwave mediated reactions) were failed to give desired reaction products. The absence of non-specific DNA bands with or without enzyme showed that microwave neither induces non-specific nuclease activity nor cause fragmentation of DNA by the invented reaction conditions.

In still another embodiment of the present invention, Stability of HindIII on microwave exposure for 0-130 seconds at highest power level (700 watts) was studied. It was observed that HindIII in its dilution buffer containing 50% glycerol (v/v) was found to loose its activity when irradiated for 0-130 seconds at 700watts.

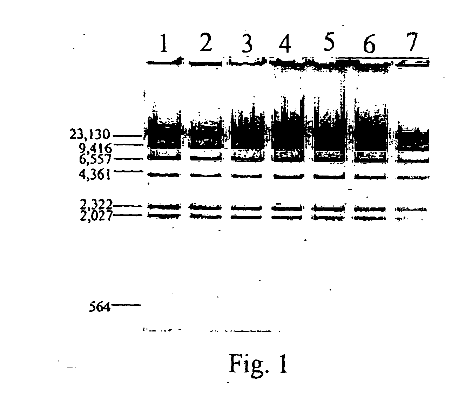

In still another embodiment of the present invention, In contrast, the same enzyme (HindIII) in its digestion buffer (overall glycerol content of the reaction mixture was 6.25% v/v) was found to be stable when exposed to microwaves for 0-130 seconds at 700watts (example 1, fig. 1).

In still another embodiment of the present invention, we have found that restriction endonuclease digestion can be carried out by the invented procedure that is by continuous microwave exposure to the reaction mixture for a short time without any break at the highest power level. This is in contrary to the reported method where enzyme was found to loose its activity at high power level.

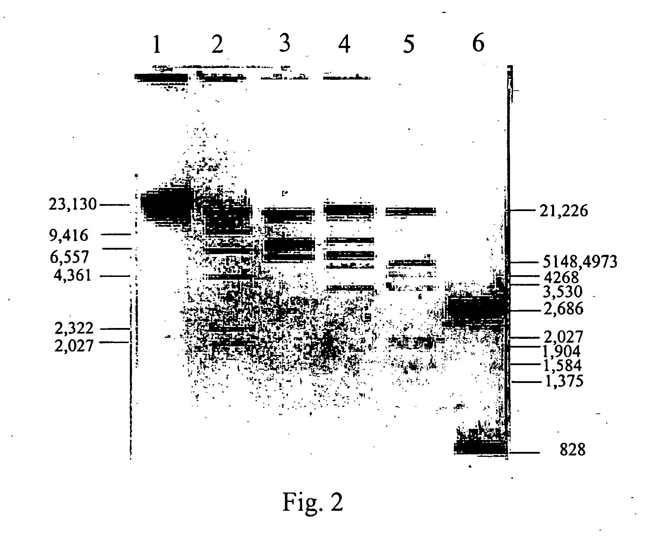

In still another embodiment of the present invention, the procedure resulted in a highly selective digestion of nucleic acids with high specificity (digested DNA were religatable) in considerable less time. Endonuclease activities of the restriction enzymes such as BamHI, EcoRI, HindIII, HaeIII, BglII, Pstl and BstEII were enhanced when the reaction mixture containing substrate DNA, enzyme and compatible buffer were exposed to microwaves at the highest power level. In a typical experiment, in 20 µl reaction volume one µg of lambda DNA was digested completely by 5-20 units (as defined by New England Biolabs) of each restriction enzyme when exposed to microwaves for 30-70 seconds (example 2, fig. 2). The double digestion of the substrate DNA with two enzymes such as EcoRI and Hind III were also found to be completed within the same time when exposed to microwaves. Another advantage of the restriction endonuclease digestion by the invented procedure is that the method is quite uniform over a wide range of restriction endonucleases as well as substrate DNAs. Substrate DNA includes lambda phage, baculovirurs, plasmid, plant and mammalian. The site-specific cleavage of substrate DNA by the restriction enzymes following the invented procedure was verified by ligation reactions carried out by the invented and conventional procedures.

In still another embodiment of the present invention, staggered cut DNA fragments produced by Hind III and blunt ended DNA fragments produced by Hae III were ligated completely by T4 DNA ligase in 10 to 15 seconds by microwave irradiation. Control experiment is carried out at 16°C for 8 hours as per conventional procedure.

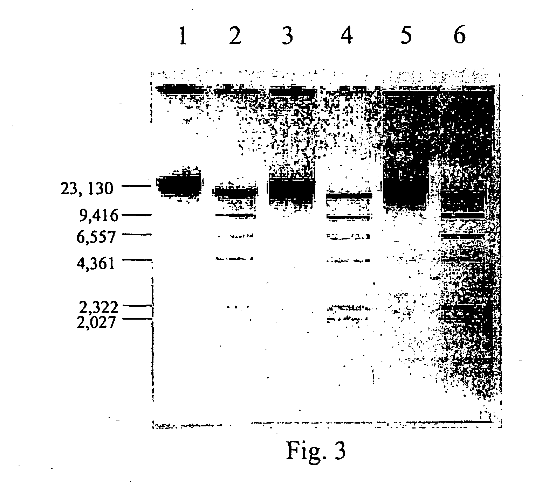

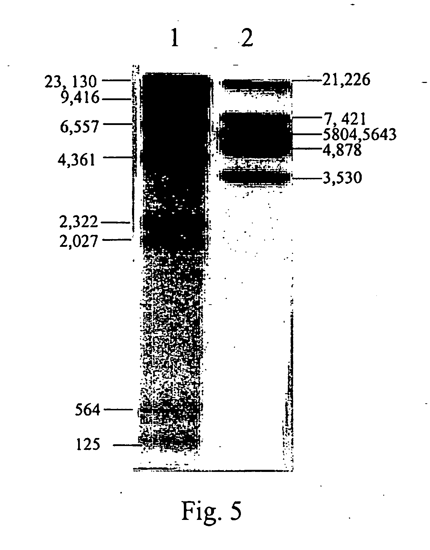

In still another embodiment of the present invention, restriction endonuclease produced DNA fragments (generated by conventional and invented procedures) were used as the substrate for ligation reactions and the ligated DNAs generated the same specific fragments when subjected to restriction endonuclease digestion with the same enzyme again (example 3, fig. 3).

In still another embodiment of the present invention, modified nucleic acids namely dephosphorylated, phosphorylated and ligated DNAs are important tools in molecular biology. All these products were obtained in sufficient quantities within 10-60 seconds of microwave exposure of the reaction mixture, a remarkable reduction in reaction time in comparison to conventional procedure. Thus, dephosphorylation of terminal phosphate groups of restriction endonuclease generated DNA fragments was carried out using calf intestinal alkaline phosphatase (CIP) in the microwave oven for different duration of time ranging from 20-50 seconds. The microwave mediated reaction products (dephosphorylated DNA) were verified when it failed to ligate by T4 DNA ligase but was kinasable by T4 polynucleotide kinase (example 4). Dephosphorylated restriction endonuclease cleaved DNA fragments were phosphorylated by T4 polynucleotide kinase (TPNK) and [γ ³² P] ATP in the microwave oven within 10 to 50 seconds. Phosphorylation of DNA is estimated by both trichloroacetic acid precipitable counts and autoradiography. The extent of phosphorylation obtained in 30 seconds microwave reaction is equal to that obtained in the conventional reaction carried out at 37°C for 20 minutes (example 5).

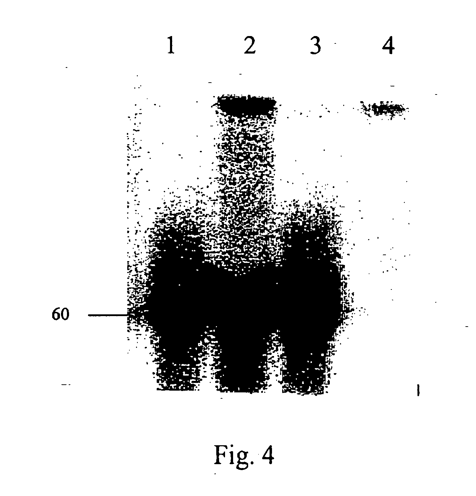

In still another embodiment of the present invention, synthetic oligonucleotides were also used as a substrate for kination reaction and later dephosphorylation reaction in the microwave oven. Both the reactions were found to occur with same efficiency as in the case of nucleic acid (example 5 b, Fig. 4).

In still another embodiment of the present invention, the enzyme-catalyzed *in vitro* syntheses in the invented procedure include DNA template directed DNA and RNA synthesis and RNA template directed DNA synthesis (reverse transcription).

In still another embodiment of the present invention, DNA dependent DNA synthesis catalyzed by *E.coli* DNA polymerase I was carried out by microwave irradiation using nicked DNA template (nick translation) and staggered cut restriction endonuclease fragments having 3' recessed ends as templates. Nick translation reactions were carried out in the microwave oven for different duration of time. DNA synthesis was monitored by incorporation of [α³²P] dCTP in trichloroacetic acid (TCA) precipitable counts. The TCA precipitable counts per microgram of nicked DNA in 45 seconds microwave reaction was found to be same as that of conventional reaction, carried out at 16°C for 60 minutes. However, that obtained in 30 seconds microwave reaction was found to be less whereas in 60 seconds it gave better result than the conventional reaction (example 6).

In still another embodiment of the present invention, the fidelity of DNA synthesized in nick translation reaction by the invented procedure was further verified by Southern hybridization with template DNA (un-nicked). Blunt ended DNA was produced in 20 seconds when the reaction mixture containing Klenow fragment of *E. coli.* DNA polymerase I and DNA templates having 3'recessed end is exposed to microwaves. The extent of DNA synthesis in 20 seconds microwave irradiation was found to be same as in the control experiment carried out at 37°C for 30 minutes. Microwave exposure for less time shows incomplete result whereas higher time shows same or little better results. The DNA synthesized under the microwaves were ascertained by (i) incorporation of [α³² P] dCTP, (ii) autoradiography and (iii) ligation to blunt ended vector DNA (example 7; fig. 5, lane 1).

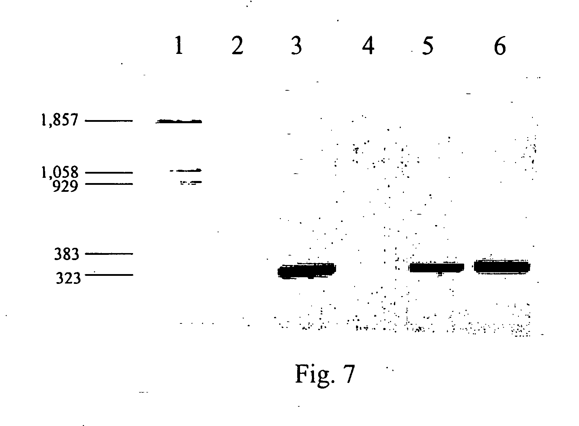

In still another embodiment of the present invention, catalytic activities of RNA polymerase and reverse transcriptase were also found to accelerate under the influence of microwaves. Thus, RNA directed DNA (cDNA) synthesis catalyzed by avian myeloblastosis virus reverse transcriptase (AMV-RT) was carried out with two template RNAs and template specific primers by microwave irradiation in different time. The optimum exposure time was found 50 seconds, which gave the same amount of synthesis as in the control reaction, carried out by the conventional procedure at 48°C for 45 minutes. The appreciable extent of reaction occurred even in 10 seconds of microwave exposure. Synthesis of cDNAs was monitored after amplification by Tfl polymerase in reverse transcriptase-polymerase chain reaction (RT-PCR) (example 8, fig. 6 and example 9, fig. 7).

In still another embodiment of the present invention, DNA directed RNA synthesis (transcription) requires specific DNA sequence (promoter) for binding of RNA polymerase to template DNA to initiate transcription. The *in vitro* synthesis of RNA catalyzed by T7 RNA polymerases were carried out using pSPT 18 and pSPT 19- neo DNA templates respectively (Boehringer Mannheim Germany, Cat No. 999644). Products obtained by microwave irradiation of the reaction mixtures for different time period ranging from 5-20 seconds were assayed by [α ³²P] UTP incorporation and the synthesis of the transcript of specific size. Trichloroacetic acid precipitable counts obtained in 5 seconds microwave reaction was found to be almost equal to that obtained in conventional reaction carried out at 37°C for 20 minutes.

In still another embodiment of the present invention, all the reactions were carried out in domestic microwave oven (BPL, India), operating at a frequency of around 2450 MHz with a maximum power out put of around 700w. All the reactions were conducted in the microwave oven at the highest power setting (level 10), that means the magnetron duty cycle of 100% of the out put power of 700watts.

In still another embodiment of the present invention, reactions were set up by sequential mixing of buffer, substrate (s) and enzyme in definite volume as followed in the conventional protocol. In still another embodiment of the present invention, all these enzyme- catalyzed reactions were carried out in the pH and the ionic environment that were used in the conventional reactions. Reaction mixture was taken in the eppendorf tube and was placed at the center of the rotating disk of the microwave oven. Reaction mixture was exposed to microwaves for a period preferably of 5-120 seconds. During the microwave irradiation the eppendorf tube was kept uncapped. Reactions were stopped immediately after the microwave irradiation was over by adding ethylenediamine tetraacetate (EDTA, pH 8.0) and /or heating at 75°C for 3-15 minutes. The reaction products were analyzed by conventional procedures such as agarose gel electrophoresis, autoradiography, trichloroacetic acid (TCA) precipitable counts, ligation, kination, hybridization, and transformation. The results of all the reactions done by the invented procedures were compared with that obtained in the conventional reaction carried out by incubating the reaction mixture in the water bath kept at temperature optima of the enzyme.

The present invention is a distinct instance of improvement for the site specific cleavage, modification and synthesis of nucleic acids over the existing methods known till date.

The following examples describe the details of the invention and are produced by way of illustration only and therefore should not be construed to limit the scope of the invention. It is contemplated that the invention as hereinafter claimed will be subject to various modifications within the scope thereof.

### Example-1

### Stability of HindIII in its digestion buffer after exposure to microwaves at 700 watts.

16 µl aliquotes of HindIII was taken in the eppendorf tubes after diluting 5 µl (100 units) of it to 144 µl with a mixture of 16 µl 10x HindIII digestion buffer (100 mM Tris HCl, pH 7.9, 500 mM NaCl, 100 mM MgCl₂, 10 mM DTT) and 107 µl water. Each aliquot was exposed to microwaves at 700 watts (power level 10) for different periods: 0, 30, 50, 70, 90, 110 and 130 seconds. Each irradiated enzyme was assayed by digestion of lambda DNA by conventional and microwave mediated reactions.

Digestion with the irradiated enzyme was carried out after adding 4 µl (2.0 µg) of lambda DNA in each tube. Half of their contents was taken in an eppendorf tube and incubated at 37°C for 4 hours as in the conventional procedure.

In microwave mediated reaction, the remaining half was spotted on a piece of parafilm, placed it at the centre of the microwave oven and irradiated by microwaves at 700 watts for 50 seconds.

All reaction products were run on 1.0 % agarose gel where 1.0 µg of digested DNA was loaded in each well (fig. 1).

### Example-2

### Restriction endonuclease digestions of DNA by microwave irradiation at 700 watts.

All the digestions were carried out at a time by spotting the reaction mixtures on a parafilm in the microwave oven for 50 seconds. Digestions of lambda DNA by Hind III, BamHI, EcoRI, double digestions of lambda DNA by EcoRI and HindIIII and digestion of a pea lectin cDNA clone at the Pstl site of PUC19 vector by PstI were carried out using 2.0 µg of DNA, 1.0 µl of each enzyme in their respective digestion buffer in 20.0 µl reaction volume. Double-digestion with HindIII and EcoRI was performed in EcoRI digestion buffer. In the control reaction 2.0 µg lambda DNA in 20.0 µl HindIII digestion buffer without enzyme was used. Each µl of the enzyme contained either 20.0 (HindIII and EcoRI) or 10.0 (BamHI and pstI) units. In each lane of the 1.0 % agarose gel 1.0 µg DNA was loaded (fig. 2).

### Example-3

### Digestion of lambda DNA by HindIII followed by ligation reaction by T4 DNA ligase by microwave irradiation

HindIII fragments for ligation reactions were prepared by digesting 8.0 µg of DNA in 160.0 µl reaction volume containing 5.0 µl (100 units) HindIII and 16.0 µl 10 X HindIII digestion buffer in a 1.5- ml eppendorf tube for 50 seconds in the microwave oven.

Ligation reaction mixture of 20.0 µl containing 6.0 µg HindIII fragments, 2.0 µl of 10 X ligation buffer (50 mM Tris-HCl (pH 7.5), 10 mM MgCl₂, 10 mM DDT, 1 mM ATP and 25 µg/ml BSA) and 20 cohesive units of T4 DNA ligase (New England Biolab.Inc.USA) were taken in two 0.5 ml eppendorf tubes in 10.0 µl aliquots. One of the tubes containing reaction mixture was exposed to microwaves for 20 seconds and the other tube was kept at 37°C for 45 minutes. The reactions were terminated by heating at 65°C for 5 minutes. Half of the DNA (1.5 µg) from each of the eppendorf tube was used for re-cut with HindIII by microwave irradiation as described above. The remaining half was used to ascertain the generation of ligated fragments by agarose gel electrophoresis.

In control experiment, uncut lambda DNA (1.0 µg) was irradiated by microwaves for 50 seconds in HindIII digestion buffer at 700 watts to find out nonspecific cleavage, if any (fig. 3).

### Example-4

### Dephosphorylation reaction by alkaline phosphatase.

Dephosphorylation of 5' phosphate groups of lambda EcoRI fragments was carried out using 5 units of calf intestinal alkaline phosphatase (Boehringer Mannheim) in a 20 µl reaction volume containing 1.0 µg DNA, 10 mM Tris-HCl (pH 7.9), 10 mM MgCl₂ 50 mM NaCl and 1 mM DTT in the microwave oven After 30 seconds of microwave exposure the reaction was terminated by the addition of 2 µl of 0.5 mM EDTA (pH 8.0) followed by incubation at 75°C for 15 minutes. DNA was deproteinized by phenolchloroform extraction and finally precipitated with ethanol. The phosphatase treated DNA fragments thus obtained was found not ligatable by T4 DNA ligase but efficiently kinased by polynucleotide kinase confirming the efficiency of dephosphorylation reaction in 30 seconds.

### Example-5

### Kination reaction by T4 polynucleotide kinase.

a) Fragmentation of lambda DNA by EcoRI was done in 50 seconds by microwave irradiation as described in example 2.
   Dephosphorylation reaction by CIP was carried out in a 20.0 µl reaction volume in 1.5 ml eppendorf tube for 30 seconds in the microwave oven using 1.0 µg DNA and 1.0 unit CIP as described above.
   Phosphorylation of 5' hydroxyl groups of dephosphorylated EcoRI fragments of 0.5 µg lambda DNA was carried out by spotting 20 µl reaction mixture comprising T4 polynucleotide kinase (Bangalore Genei), 70 mM Tris-HCl (pH 7.6), 10 mM MgCl₂, 5 mM DTT and 20 µCi [γ³² P] ATP (Sp.activity. >4000 Ci/mmole) on parafilm and exposing it to microwaves for 20 seconds. The reaction was terminated by adding EDTA (pH 8.0) to 5 mM (final concentration) followed by heating to 65°C for 10 minutes. Unincorporated radioactivity was removed by spin-column Sephadex G-50 gel filtration. Phosphorylations of DNA fragments are verified by autoradiography by loading reaction volume equivalent to 0.1µl.
b) Phosphorylation of 5' hydroxyl groups of 0.1 µg 60 mer oligonucleotide (5' - CCCCGCCCCGCG - 3') ₅ by T4 polynucleotide kinase was carried out by microwave irradiation in different time periods (0, 50, 30 and 20 seconds) as above (fig. 4)

### Example-6

### DNA synthesis by E.coli polymerase I.

DNA synthesis reactions catalyzed by *E.coli.* DNA polymerase I was carried out in 50 µl reaction volumes containing 1 µg nicked lambda phage DNA or *Autographa Californica nucleopolyhedrosis* virus (*Ac*NPV) DNA template (treated with 5 femtogram of Dnase I), 5 units of *E.coli* DNA polymerase I (NEB), 50 mM Tris-HCl (pH 7.5), 10 mM MgS0₄, 1 mM dithiothreitol (DTT), 50 µg/ml bovine serum albumin (BSA, pentax fraction V), unlabeled dATP, dTTP, dGTP (1 nM each), 100 µCi [α³² P] dCTP (Sp.activity. >4000 Ci/mmole) in the microwave oven for different duration of time. The reactions were stopped by the addition of 4µl of 0.25 M EDTA (pH8.0) after the exposure to microwaves. DNA synthesis with each DNA template was measured by trichloroacetic acid (TCA) precipitation assays after the removal of unincorporated radionucleotide by centrifugation through a small column of Sephadex G-50. The amount of DNA synthesized in 45-second microwave reaction was equal to that synthesized in the conventional reaction carried out at 16°C for 60 minutes. The labeled DNAs synthesized by the invented procedure efficiently hybridized with the unlabeled restriction endonuclease digests of the template DNAs.

### Example 7

### DNA synthesis by Klenow fragment E.coli polymerase I.

DNA synthesis reactions catalyzed by the Klenow fragment of *E.coli.* DNA polymerase I were done in 20 µl reaction volume containing 1µg of HindIII fragments of lambda phage DNA (produced by microwave irradiation as described in example 1), 1 µl (8 units) of Klenow fragment of *E*. *coli* DNA polymerase I, 50 mM Tris HCl, pH 7.2, 10 mM MgSO₄, 1 mM DTT, 50 µg/ml BSA, 4 pmoles of [α³² P] dCPT (Sp.act. >4000 µCi /mmole) and 2 n moles of each dTTP, dATP, dGTP in the microwave oven. The reaction mixture was spotted on parafilm and was exposed to microwaves for 20 seconds after which the reaction is stopped by adding 4 µl of 0.25 M EDTA (pH 8.0). Unincorporated nucleotides are removed by Sephadex G-50 spin column when radionucleotide is used. The filling of the 3' recessed ends of template DNA by DNA synthesis is measured by TCA precipitable counts and the specificity of the DNA synthesis is ascertained by autoradiography and ligation to blunt ended vector DNA. The extent of DNA synthesized by the invented microwave reaction was equal to that obtained by the conventional method at 37°C for 30 minutes (Fig. 5, lane 1)

### Example-8

### Microwave mediated E-selectin mRNA template directed cDNA synthesis by AMV-RTase.

Three different sets of experiment were made for the synthesis of cDNA catalyzed by avian myeloblastosis virus reverse transcriptase (AMV-Rtase) using E-selectin [Tucker et al. (1991) J.Biol. Chem., 266, 2466-2473] with specific forward primers by exposing the reaction mixture to microwaves for 10, 30 and 50 seconds respectively. The cDNA of specific size was identified by agarose gel electrophoresis after amplification of first strand cDNA reaction product following the addition of template specific reverse and forward primers and Tfl DNA polymerase. Forward and reverse primer used for E-selectin mRNA templates were 5-GAGTGGGCATGTGGAATGATG-3 and 3'-GGTCTCGGAAGTCACATGGA-5' respectively. The second strand cDNA synthesis and PCR amplification after inactivation of AMV reverse transcriptase (94°C for 2 min.) were done in 40 cycles (94°C for 30 seconds/ denaturation, 60°C for 1 min./annealing, 68°C for 2 min./ extension). Composition of RT-PCR reaction in 50 µl reaction volume was 50 mM Tris-HCl (pH 8.3), 50 mM KCl, 10 mM MgCl₂, 0.5mM spermidine, 10mM DTT, 0.2mM dNTP (each), 1µM forward primer, 1 µm reverse primer, 1 mM MgSO₄, 5 units AMV-Rtase and 5 units Tfl DNA polymerase. The reaction was initiated by the addition of RNA. All the RT-PCR reaction components except E-selectine mRNA and its primers are procured from Promega.

Formation of amplified products of specific size, that is 501 bp with E-selectin mRNA template in both invented and conventional reactions confirmed the specific first strand cDNA synthesis with the mRNA. The amounts of amplified cDNAs synthesized in 50 seconds were more than that obtained in the conventional reaction (fig. 6)

### Example-9

### Microwave mediated synthetic mRNA template directed cDNA synthesis by AMV-RTase.

Three different sets of experiment were made for the synthesis of cDNA catalyzed by avian myeloblastosis virus reverse transcriptase (AMV-Rtase) using synthetic mRNA templates (an 1151 nt *in vitro* synthesized RNA from Promega) with specific forward primers by exposing the reaction mixture to microwaves for 10, 30 and 50 seconds respectively. The cDNA of specific size is identified by agarose gel electrophoresis after amplification of first strand cDNA reaction product following the addition of template specific reverse and forward primers and Tfl DNA polymerase. Forward and reverse primers used for synthetic mRNA templates were 5'GCCATTCTCACCGGATTCAGTCGTC-3' and 3' GACTGAACTGCCGCCGA-5' respectively. Formation of amplified products of specific size, that is 323 bp with synthetic mRNA template in both invented and conventional reactions confirmed the specific first strand cDNA synthesis with the mRNA. The amounts of amplified cDNAs synthesized in 50 seconds were equal to that obtained in the conventional reaction.

The second strand cDNA synthesis and PCR amplification after inactivation of AMV reverse transcriptase (94°C for 2 min.) were done in 40 cycles (94°C for 30 seconds/ denaturation, 60°C for 1 min./annealing, 68°C for 2 min./ extension). Composition of RT-PCR reaction in 50 µl reaction volume was 50 mM Tris-HCl (pH 8.3), 50 mM KCl, 10 mM MgCl₂, 0.5mM spermidine, 10mM DTT, 0.2mM dNTP (each), 1 µM forward primer, 1 µM reverse primer, 1 mM MgSO₄, 5 units AMV-Rtase and 5 units Tfl DNA polymerase. The reaction was initiated by the addition of RNA. All the RT-PCR reaction components are procured from Promega (Fig.7).

### Example-10

### RNA synthesis by T7 RNA polymerase.

RNA synthesis catalyzed by SP6 and T7 RNA polymerase was carried out using EcoRl cleaved pSPT18-neo and pSPT19-neo DNA (Boehringer Mannheim, Germany) respectively in 20 µl reaction volume. Each reaction mixture contained 0.5 µg DNA template, 0.5 mM each of ATP, GTP, CTP, UTP, 40 mM Tris-HCl (pH 7.9), 6 mM MgCl₂, 2 mM spermidine, 10 mM DTT. For the labeled synthesis 0.5 mM UTP is replaced by 50µ Ci [α³² P] UTP (>400 Ci /mmol). The reaction mixture was exposed to microwave irradiation for 20 seconds after which the reaction was terminated by adding 2 µl of 0.25 M EDTA (pH 8.0). RNA synthesis was measured by incorporation of [α³² P] UTP in the TCA precipitable counts. TCA precipitable RNA synthesized by the invented procedure was found to be more than the conventional procedure carried out at 37°C for 45 minutes. The size of RNA synthesized using cold NTPs was determined by formaldehyde-agarose gel and was found to be identical to that obtained in conventional reactions.

### Industrial applicability

This invention useful for rapid and efficient method for the preparation of products produced by enzyme-catalyzed reactions on brief and uninterrupted microwave exposure. The invented procedure can be adapted in biotechnology, more particularly in molecular biology, industrial processing and sewage treatment. The invented procedure will also be suitable for automation in the related field.

The microwave-based procedure of the present invention is particularly useful for rapid site-specific cleavage of DNA, modification of DNA and oligonucleotide, and, *in vitro* synthesis of nucleic acids. The reactions include restriction endonuclease digestion of DNA, ligation, dephosphorylation, kination, and *in vitro* synthesis of nucleic acids. The products obtained therein are key to the molecular biology and are required for genome mapping, DNA amplification, DNA sequencing, gene expression, desired product purification, desired product isolation, manipulation of the gene in recombinant DNA technology etc. The present invention is suitable for automation in molecular biology.

### Advantages

1. The drastic reduction in the reaction time for the site specific cleavage, modification of DNA and oligonucleotide, and synthesis of nucleic acids is the main advantage of the present invention. The reaction time is reduced to 5-70 seconds for restriction endonuclease digestion, ligation, dephosphorylation, phosphorylation (kination), reverse transcription, *in vitro* DNA synthesis and *in vitro* RNA synthesis.
2. Almost all the enzymatic reactions used in molecular biology as well as in other field can be done by the invented procedure in an extraordinary short period, saving a lot of precious time and thus making the procedure economical and versatile.
3. The reaction conditions are very simple and no need of any costly equipment.
4. The invented procedure can be used for automation in molecular biology.
5. The invented procedure can be used in biotechnology industry involving enzymatic reactions.

## Claims

1. A simple, efficient, and accelerated method for enzyme-catalyzed in *vitr*o modification and synthesis of nucleic acid using uninterrupted and brief microwave irradiation., said method comprising steps of:
(a) providing a reaction mixture comprising (i) an enzyme selected from the group consisting of restriction endonuclease, ligase, phosphatase, kinase, reverse transcriptase, DNA polymerase and RNA polymerase (ii) a biomolecule selected from DNA, RNA or oligonucleotides, to act as a substrate for the selected enzyme, (iii) suitable buffer for specific enzyme and (iv) other ingredients, in a small eppendorf tube or on a small piece of parafilm,
(b) placing the said eppendorf tube or parafilm piece, loaded with the reaction mixture, inside a microwave oven,
(c) irradiating the said eppendorf tube or parafilm by microwaves continuously at a frequency ranging between 2300 to 2500 MHz with the power output ranging between 600 to 900 watts,
(d) for a period ranging from 5 to 120 seconds,
(e) terminating the reaction by adding ethylenediamine tetra acetate (EDTA) and/ or heating in a water bath at 75°C for 3-15 minutes followed by the addition of gel loading dye,
(f) analyzing the reaction product (s) by agarose gel electrophoresis, autoradiography, radioactive counts and other techniques as used in the conventional procedures.

2. A method as claimed in claim 1, wherein nucleic acid is selected from DNA, RNA, and oligonucleotide.

3. A method as claimed in claim 1, wherein other ingredients used are selected from a group comprising MgCl₂, MgSO₄, NaCl, KCl, CH₃COOK, ethylenediamine tetra acetate, dithiothreitol, spermidine, BSA, triton x-100, nucleotides, template DNA, template RNA, and oligonucleotide primers.

4. A method as claimed in claim 1, wherein restriction endonuclease is selected from Hind III, BamHI, EcoRI, Hae III, Bgl II, Pst I, Bst E II, and Bst NI.

5. A method as claimed in claim 1, wherein ligase used is T4 DNA ligase.

6. A method as claimed in claim 1, wherein kinase used is T4 polynucleotide kinase (TPNK).

7. A method as claimed in claim 1, wherein phosphatase used is calf intestinal alkaline phosphatase (CIP).

8. A method as claimed in claim 1, wherein polymerase is selected from the group consisting of E.coli. DNA polymerase I, Klenow fragment of E.coli. DNA polymerase I, T7 RNA polymerase, and SP6 RNA polymerase.

9. A method as claimed in claim 1, wherein reverse transcriptase used is avian myeloblastosis virus-reverse transcriptase (AMV-RT).

10. A method as claimed in claim 1, wherein DNA used is selected from the group comprising genomic DNA, lambda phage DNA; plasmid DNA, baculovirus DNA, plant DNA, and mammalian DNA.

11. A method as claimed in claim 1, wherein more than one restriction endonuclease is used for double digestion of nucleic acid.

12. A method as claimed in claim 11, wherein restriction endonucleases used are EcoRI and Hind III for double digestion.

13. A method as claimed in claim 1, wherein microwave irradiation can be carried out in any apparatus or chamber where microwave can be generated.

14. A method as claimed in claim 1, wherein the said method can be partially of fully automated by a specially designed apparatus.

## Patentansprüche

1. Einfaches, effizientes und beschleunigtes Verfahren zur Enzym-katalysierten *in vitro-*Modifizierung und Synthese von Nukleinsäure unter Verwendung einer nicht-unterbrochenen und kurzen Mikrowellenbestrahlung, wobei das Verfahren folgende Schritte umfasst:
(a) Bereitstellen einer Reaktionsmischung, umfassend (i) ein Enzym, ausgewählt aus der Gruppe bestehend aus Restriktionsendonuklease, Ligase, Phosphatase, Kinase, reverse Transkriptase, DNA-Polymerase und RNA-Polymerase, (ii) ein Biomolekül, ausgewählt aus DNA, RNA oder Oligonukleotiden, um als ein Substrat für das ausgewählte Enzym zu wirken, (iii) einen geeigneten Puffer für das spezielle Enzym und (iv) andere Inhaltsstoffe, in einem kleinen Eppendorf-Reaktionsgefäß oder auf einem kleinen Stück Parafilm,
(b) Platzieren des mit der Reaktionsmischung beladenen Eppendorf-Reaktionsgefäßes oder Parafilm-Stücks in einen Mikrowellenherd,
(c) Bestrahlen des Eppendorf-Reaktionsgefäßes oder Parafilms kontinuierlich durch Mikrowellen mit einer Frequenz, welche von 2300 bis 2500 MHz eingeschlossen reicht, wobei die Leistungsabgabe von 600 bis 900 Watt eingeschlossen reicht,
(d) für eine Zeitspanne, die von 5 bis 120 s eingeschlossen reicht,
(e) Beenden der Reaktion durch Zugeben von Ethylendiamintetraacetat (EDTA) und/oder Erwärmen in einem Wasserbad bei 75°C für 3-15 min, gefolgt von der Zugabe von Gelauftragsfarbstoff,
(f) Analysieren des Reaktionsprodukts (s) durch Agarose-Gelelektrophorese, Autoradiographie, Radioaktivitätszählungen und andere Techniken, wie sie in den herkömmlichen Verfahren verwendet werden.

2. Verfahren nach Anspruch 1, wobei die Nukleinsäure aus DNA, RNA und Oligonukleotiden ausgewählt wird.

3. Verfahren nach Anspruch 1, wobei andere Inhaltsstoffe, die verwendet werden, aus einer Gruppe, umfassend MgCl₂, MgSO₄, NaCl, KCI, CH₃COOK, Ethylendiamintetraacetat, Dithiothreitol, Spermidin, BSA (Rinderserumalbumin), Triton X-100, Nukleotide, Matrizen-DNA, Matrizen-RNA und Oligonukleotid-Primer, ausgewählt werden.

4. Verfahren nach Anspruch 1, wobei die Restriktionsendonuklease aus Hindlll, BamH1, EcoR1, Haelll, Bglll, Pstl, BstEll und BstNl ausgewählt wird.

5. Verfahren nach Anspruch 1, wobei die verwendete Ligase T4-DNA-Ligase ist.

6. Verfahren nach Anspruch 1, wobei die verwendete Kinase T4-Polynukleotidkinase (TPNK) ist.

7. Verfahren nach Anspruch 1, wobei die verwendete Phosphatase alkalische Phosphatase aus Kälberdarm (CIP) ist.

8. Verfahren nach Anspruch 1, wobei die Polymerase aus der Gruppe bestehend aus E. coli-DNA-Polymerase I, dem Klenow-Fragment von E. coli-DNA-Polymerase I, T7-RNA-Polymerase und SP6-RNA-Polymerase ausgewählt wird.

9. Verfahren nach Anspruch 1, wobei die verwendete reverse Transkriptase die reverse Transkriptase des Vögel-Myeloblastosevirus (AMV-RT) ist.

10. Verfahren nach Anspruch 1, wobei die verwendete DNA aus der Gruppe, umfassend genomische DNA, Lambda-Phagen-DNA, Plasmid-DNA, Baculovirus-DNA, Pflanzen-DNA und Säugetier-DNA, ausgewählt wird.

11. Verfahren nach Anspruch 1, wobei mehr als eine Restriktionsendonuklease für einen doppelten Verdau von Nukleinsäure verwendet wird.

12. Verfahren nach Anspruch 11, wobei die verwendeten Restriktionsendonukleasen EcoRI und HindIII für einen doppelten Verdau sind.

13. Verfahren nach Anspruch 1, wobei die Mikrowellen-Bestrahlung in einem jeglichen Gerät oder einer jeglichen Kammer, wo Mikrowellen erzeugt werden können, ausgeführt werden kann.

14. Verfahren nach Anspruch 1, wobei das Verfahren teilweise oder vollständig durch ein speziell gestaltetes Gerät automatisiert werden kann.

## Revendications

1. Procédé simple, efficace et accéléré pour la modification et la synthèse in vitro catalysée par enzyme d'acide nucléique en utilisant une irradiation ininterrompue et brève aux micro-ondes, ledit procédé comprenant les étapes de:
(a) placement d'un mélange réactionnel contenant (i) un enzyme sélectionné parmi le groupe composé d'endonucléase de restriction, ligase, phosphatase, kinase, transcriptase reverse, polymérase d'ADN et polymérase d'ARN, (ii) d'une biomolécule sélectionnée parmi l'ADN, l'ARN ou les oligonucléotides pour faire office de substrat pour l'enzyme sélectionné, (iii) d'un tampon approprié pour un enzyme spécifique et (iv) d'autres ingrédients dans un petit tube Eppendorf ou sur un petit morceau de parafilm,
(b) placement dudit tube Eppendorf ou morceau de parafilm chargé du mélange réactionnel à l'intérieur d'un four à micro-ondes,
(c) irradiation dudit tube Eppendorf ou parafilm en continu aux micro-ondes à une fréquence allant de 2300 à 2500 MHz avec une puissance fournie allant de 600 à 900 watts,
(d) pendant une durée allant de 5 à 120 secondes,
(e) achèvement de la réaction en ajoutant de l'éthylènediamine tétra acétate (EDTA) et/ou en chauffant dans un bain aqueux à 75° C pendant 3 à 15 minutes, ce qui est suivi de l'addition de colorant de charge de gel,
(f) analyse du(des) produit(s) réactionnel(s) par électrophorèse au gel d'agarose, autoradiographie, comptes de coups par minute radioactifs et autres techniques telles qu'utilisées dans les procédures conventionnelles.

2. Procédé selon la revendication 1, dans lequel l'acide nucléique est sélectionné parmi les éléments ADN, ARN et oligonucléotide.

3. Procédé selon la revendication 1, dans lequel d'autres ingrédients utilisés sont sélectionnés parmi un groupe comprenant les éléments MgCl₂, MgSO₄, NaCl, KCl, CH₃COOK, éthylènediamine tétra acétate, dithithréitol, spermidine, BSA, triton x-100, nucléotides, matrice d'ADN, matrice d'ARN et amorces d'oligonucléotide.

4. Procédé selon la revendication 1, dans lequel l'endonucléase de restriction est sélectionnée parmi les éléments HindIII, BamHi, EcoRI, HaeIII, BglII, PstI, BstEII et BstNI.

5. Procédé selon la revendication 1, dans lequel la ligase utilisée est de la ligase d'ADN T4.

6. Procédé selon la revendication 1, dans lequel la kinase utilisée est de la kinase de polynucléotide T4 (TPNK).

7. Procédé selon la revendication 1, dans lequel la phosphatase utilisée est de la phosphatase alcaline d'intestin de veau (CIP).

8. Procédé selon la revendication 1, dans lequel la polymérase est sélectionnée parmi le groupe composé d'E.coli, de polymérase I d'ADN, d'un fragment Klenow de polymérase I d'ADN d'E.coli, de polymérase d'ARN T7 et de polymérase d'ARN SP6.

9. Procédé selon la revendication 1, dans lequel la transcriptase reverse utilisée est de la transcriptase reverse de virus de myéloleucose aviaire (AMV-RT).

10. Procédé selon la revendication 1, dans lequel l'ADN utilisé est sélectionné parmi le groupe composé de l'ADN génomique, l'ADN de phage lambda, l'ADN de plasmide, l'ADN de baculovirus, l'ADN de végétal et l'ADN de mammifère.

11. Procédé selon la revendication 1, dans lequel plus d'une endonucléase de restriction est utilisée pour la double digestion d'acide nucléique.

12. Procédé selon la revendication 11, dans lequel les endonucléases de restriction utilisées sont l'EcoRI et l'HindIII pour la double digestion.

13. Procédé selon la revendication 1, dans lequel l'irradiation aux micro-ondes peut être réalisée dans tout appareil ou toute chambre où des micro-ondes peuvent être générées.

14. Procédé selon la revendication 1, dans lequel ledit procédé peut être partiellement ou totalement automatisé au moyen d'un appareil conçu spécialement.


### SEQUENCE LISTING

<110> Council of Scientific and Industrial Research
<120> A simple, efficient, and accelerated method for enzyme-catalyzed in vitro modification and synthesis of nucleic acid using microwave irradiation.
<130> PCT 216
<140> PCT/IB02/02766
   <141> 2002-07-04
<160> 5
<170> Patentln version 3.1
<210> 1
   <211> 12
   <212> DNA
   <213> Artificial Sequence
<220>
   <223> oligonucleotide used for kination by T4 polynucleotide kinase
<400> 1
   ccccgccccg cg 12
<210> 2
   <211> 21
   <212> DNA
   <213> Artificial Sequence
<220>
   <223 > Forward primer for E-selectin mRNA templates
<400> 2
   gagtgggcat gtggaatgat g 21
<210> 3
   <211> 20
   <212> DNA
   <213> Artificial Sequence
<220>
   <223> Reverse primer used for E-selectin mRNA templates
<400> 3
   ggtctcggaa gtcacatgga 20
<210> 4
   <211> 25
   <212> DNA
   <213> Artificial Sequence
<220>
   <223> Forward primer used for synthetic mRNA templates
<400> 4
   gccattctca ccggattcag tcgtc 25
<210> 5
   <211> 17
   <212> DNA
   < 213 > Artificial Sequence
<220>
   <223> Reverse primer used for synthetic mRNA templates
<400> 5
   gactgaactg ccgccga 17